(19) Europäisches Patentamt
European Patent Office
Office européen des brevets

(11) **EP 4 338 657 B1**

(12) **EUROPEAN PATENT SPECIFICATION**

(45) Date of publication and mention
of the grant of the patent:
**01.04.2026 Bulletin 2026/14**

(21) Application number: **22842089.9**

(22) Date of filing: **11.07.2022**

(51) International Patent Classification (IPC):
***A61B 3/117*** *(2006.01)* ***G01N 21/21*** *(2006.01)*
***G01N 21/49*** *(2006.01)*

(52) Cooperative Patent Classification (CPC):
**G01N 21/65; A61B 3/117; A61B 5/0075;**
A61B 5/14532; G01N 2021/4726; G01N 2201/0683

(86) International application number:
**PCT/JP2022/027303**

(87) International publication number:
**WO 2023/286745 (19.01.2023 Gazette 2023/03)**

(54) **LIGHT INTENSITY MEASUREMENT SYSTEM, RAMAN SCATTERING SPECTROSCOPY SYSTEM AND LIGHT INTENSITY MEASUREMENT METHOD**

LICHTINTENSITÄTSMESSSYSTEM, RAMANSTREUUNGSSPEKTROSKOPIESYSTEM UND LICHTINTENSITÄTSMESSVERFAHREN

SYSTÈME DE MESURE D'INTENSITÉ LUMINEUSE, SYSTÈME DE SPECTROSCOPIE DE DIFFUSION RAMAN ET PROCÉDÉ DE MESURE D'INTENSITÉ LUMINEUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.07.2021 JP 2021116644**

(43) Date of publication of application:
**20.03.2024 Bulletin 2024/12**

(73) Proprietors:
• **Seed Co., Ltd.**
**Tokyo 113-8402 (JP)**
• **Keio University**
**Tokyo, 108-8345 (JP)**

(72) Inventors:
• **KINOSHITA, Taku**
**Tokyo 113-8402 (JP)**
• **SAWADA, Mio**
**Tokyo 113-8402 (JP)**
• **KURIHARA, Toshihide**
**Tokyo 160-8582 (JP)**
• **SHINOJIMA, Ari**
**Tokyo 160-8582 (JP)**

(74) Representative: **Prüfer & Partner mbB**
**Patentanwälte · Rechtsanwälte**
**Sohnckestraße 12**
**81479 München (DE)**

(56) References cited:
WO-A1-2020/195199    JP-A- H09 122 075
US-A1- 2007 004 975    US-A1- 2007 004 975
US-A1- 2010 245 764

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field

The present invention relates to a light intensity measurement system, a Raman scattered light spectroscopic measurement system, and a light intensity measurement method.

Background

There has been conventionally known a technique of analyzing substances contained in aqueous humor in a subject eye to assist in diagnosis of the subject eye. Patent Literature 1 discloses a technique of non-invasively measuring, using light, substances contained in aqueous humor of a subject eye, to ease the burden on a subject. This technique guides polarization-controlled light into aqueous humor via a cornea, and directly detects light that comes out again via the cornea. Then, by measuring a polarization state of the detected light, the technique measures concentrations of various types of substances contained in the aqueous humor.

On the other hand, in the case of guiding light into aqueous humor via a cornea, the polarization state of light is influenced by the birefringence of the cornea when the light is guided into the aqueous humor, and when the light comes out from the aqueous humor. Thus, to measure the concentration of substances contained in the aqueous humor, it is necessary to exclude the influence exerted by the birefringence of the cornea, from a measurement result. Patent Literature 1 discloses a technique of compensating for the influence exerted by the birefringence of the cornea, using a polarization plate. Patent Literature 3 discloses a contact lens having a concave inner surface that comes into contact with a subject's eye, and a convex outer surface, and is further provided with an incidenceside light guiding window that is formed to be cut from the outer surface to the inner surface side, and guides light incident from a direction approximately orthogonal to a lens geometric center axis such that the light travels along the direction approximately orthogonal to the lens geometric center axis in the aqueous humor of the subject's eye wearing the contact lens. The contact lens thereby enables non-invasive analysis of a substance contained in the aqueous humor of the subject's eye. Patent Literature 4 discloses an apparatus and a method for separately detecting and measuring specularly reflected light and diffusely reflected light following illumination of an eye by light. The apparatus and method facilitate separation of the diffusely reflected light from light specularly reflected from the eye after passing through one or more elements of the eye, for example, the cornea, lens, retinal vasculature, the nerve fiber layer and/or the photoreceptors. Furthermore, Patent Literature 5 is known which discloses a method for determining analyte concentration levels, the method including: acquiring radiation scattered off or transmitted by a target, analyzing at least a first portion of the radiation via a first technique to generate a first measurement of analyte concentration levels, and analyzing at least a second portion of the radiation via a second technique to generate a second measurement of analyte concentration levels. The method further determines analyte concentration levels based on at least one of the first measurement or the second measurement. In addition, a system for implementing the method and a probe for measuring and monitoring the analyte concentration levels are disclosed.

Citation List

Patent Literature

Patent Literature 1: WO 2005/067522 A
Patent Literature 2: US 6,885,882
Patent Literature 3: WO 2020/195199 A1
Patent Literature 4: US 2010/245764 A1
Patent Literature 5: US 2007/004975 A1

Summary

Technical Problem

Nevertheless, according to a result of anatomical research on the shape of a corneoscleral limbus that has been conducted by inventors of the present invention, and trial calculation that is based on data on the spreading of light fluxes to be guided, and an eyeball movement, in many subject eyes, to realize an optical path for measuring the concentration of substances in aqueous humor that is described in Patent Literature 1, it is necessary to control an incident angle of light with respect to a subject eye, with an error smaller than 1°, and it is considered to be difficult to realize the optical path.

The present invention has been devised in view of the foregoing, and provides a light intensity measurement system, specifically a Raman scattered light spectroscopic measurement system, and a light intensity measurement method that can non-invasively measure the concentration of substances contained in aqueous humor of a subject eye.

Solution to Problem

To solve the problem described above and to achieve the object, a light intensity measurement system, specifically a Raman scattered light spectroscopic measurement system according to a general aspect of the present invention includes: a light source unit configured to emit coherent light to aqueous humor of a subject eye from a direction approximately orthogonal to an eye axis of the subject eye; a polarization control unit that is arranged between the light source unit and the subject eye, and configured to control a polarization state of the coherent light; a measurement unit configured to measure a spectrum of Raman scattered light of the coherent light in the aqueous humor, the measurement unit being arranged in a direction approximately conforming to the eye axis of the subject eye; and a notch filter arranged between the subject eye and the measurement unit to selectively block the Rayleigh scattered light from the aqueous humor.

The Raman scattered light spectroscopic measurement system further comprises any one of the following configurations (i) to (iii):

(i) a driving unit configured to insert and remove the notch filter into and from between the subject eye and the measurement unit, wherein the polarization control unit is configured to control the polarization state of the coherent light in such a manner that a light intensity of the Rayleigh scattered light of the coherent light in the aqueous humor becomes the maximum in the spectrum measured by the measurement unit when the notch filter is not arranged on an optical path extending from the aqueous humor toward the measurement unit, and the driving unit is configured to insert the notch filter onto the optical path extending from the aqueous humor toward the measurement unit such that the measurement unit measures the spectrum of the Raman scattered light of the coherent light in the aqueous humor, the Rayleigh scattered light having the light intensity maximized by the polarization control unit;

(ii) a reflecting mirror configured to reflect the Rayleigh scattered light in the aqueous humor, a photodetector configured to detect the Rayleigh scattered light reflected by the reflecting mirror, and a slide box configured to insert and remove the reflecting mirror onto and from an optical path extending from the aqueous humor toward the measurement unit, wherein the polarization control unit is configured to control the polarization state of the coherent light in such a manner that a light intensity of the Rayleigh scattered light detected by the photodetector becomes the maximum, and the slide box is configured to remove the reflecting mirror from the optical path such that the measurement unit measures the spectrum of the Raman scattered light of the coherent light in the aqueous humor, the Rayleigh scattered light having the light intensity maximized by the polarization control unit; and

(iii) a photodetector arranged in such a manner as to form 45° with respect to an optical axis of the subject eye, the photodetector being configured to detect the Rayleigh scattered light in the aqueous humor, a light absorber configured to absorb light from the photodetector, and a slide box configured to insert and remove the photodetector onto and from an optical path extending from the aqueous humor toward the measurement unit, wherein the polarization control unit is configured to control the polarization state of the coherent light in such a manner that a light intensity of the Rayleigh scattered light detected by the photodetector becomes the maximum, and the slide box is configured to remove the photodetector from the optical path such that the measurement unit measures the spectrum of the Raman scattered light of the coherent light in the aqueous humor, the Rayleigh scattered light having the light intensity maximized by the polarization control unit.

In the Raman scattered light spectroscopic measurement system according to an embodiment of the present invention, the polarization control unit includes any one of a plurality of wave plates, a Babinet-Soleil compensator, and an acousto-optical element.

A light intensity measurement method according to another general aspect of the present invention includes: a light emission step of emitting coherent light to aqueous humor of a subject eye from a direction approximately orthogonal to an eye axis of the subject eye; a polarization control step of controlling a polarization state of the coherent light and guiding the coherent light into the subject eye in such a manner that a light intensity of the Rayleigh scattered light of the coherent light in the aqueous humor becomes the maximum in a spectrum measured by a measurement unit that is arranged in a direction approximately conforming to the eye axis of the subject eye; a filter insertion step of inserting a notch filter configured to selectively block the Rayleigh scattered light in the aqueous humor, into between the subject eye and the measurement unit; and a measurement step of measuring a spectrum of the Rayleigh scattered light in the aqueous humor by the measurement unit, the Rayleigh scattered light being through the notch filter and having the maximized light intensity.

Advantageous Effects of Invention

According to the present invention, it is possible to realize a light intensity measurement system, specifically a Raman scattered light spectroscopic measurement system, and a light intensity measurement method that can non-invasively measure the concentration of substances contained in aqueous humor of a subject eye.

Brief Description of Drawings

FIG. 1 is a schematic diagram illustrating a configuration of a Raman scattered light spectroscopic measurement system according to an embodiment of the present invention.
FIG. 2 is a flowchart illustrating processing to be executed by the Raman scattered light spectroscopic measurement system.
FIG. 3 is a schematic diagram illustrating a configuration of a Raman scattered light spectroscopic measurement system according to Modified Example 1.
FIG. 4 is a schematic diagram illustrating a configuration of a Raman scattered light spectroscopic measurement system according to Modified Example 2. Description of Embodiments

Hereinafter, the details of a light intensity measurement system, a Raman scattered light spectroscopic measurement system, and a light intensity measurement method of the present invention will be described based on an embodiment, but these are not intended to limit the present invention.

(Embodiment)

[Configuration of Raman Scattered Light Spectroscopic Measurement System]

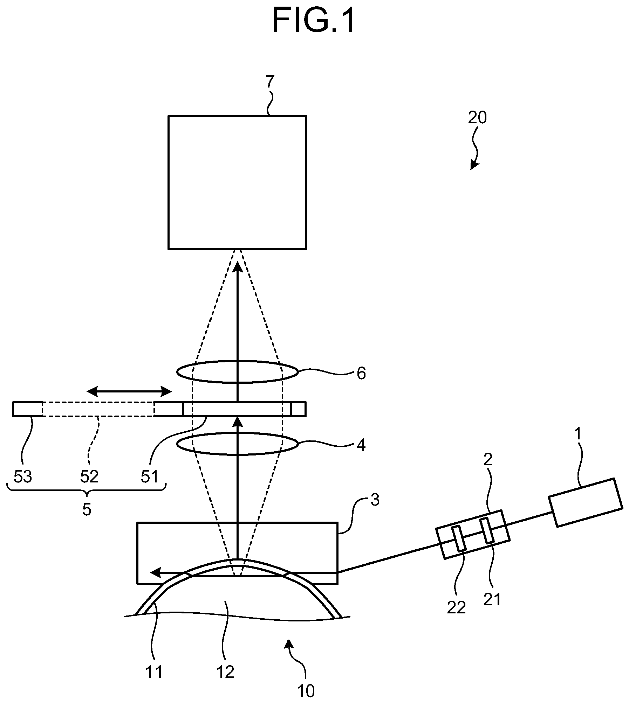

FIG. 1 is a schematic diagram illustrating a configuration of a Raman scattered light spectroscopic measurement system according to an embodiment of the present invention. A Raman scattered light spectroscopic measurement system 20 serving as a light intensity measurement system performs measurement for non-invasively analyzing, by the Raman spectroscopy, the concentration of substances contained in aqueous humor 12 existing inside a cornea 11 of a subject eye 10 being an eye of a subject.

The aqueous humor 12 is tissue fluid filling a chamber sectioned by the cornea 11, an iris, and a crystalline lens existing behind a pupil. The aqueous humor 12 is produced in a ciliary body, circulates inside the chamber via the pupil, and flows out to a vein through a Schlemm's canal from the outside of this chamber. The aqueous humor 12 is used to keep an eye pressure and maintain the shape of an anterior eye chamber of an eyeball. In addition, the aqueous humor 12 has a function of supplying energy by a glucose glycolytic system to eyeball cells such as corneal endothelial cells through which no blood vessel passes.

Although a main component of the aqueous humor 12 is water, not only glucose but also various metabolic products are dissolved in the aqueous humor 12, and it is known that there is correlation between the state of an eye disease and the concentration of a metabolic product. For example, Patent Literature 2 describes that the glucose concentration of the aqueous humor 12 changes after several minutes from a change in blood glucose concentration. There are medical needs such as the recognition of a progress level of retinopathy of diabetes by the measurement of the glucose concentration of the aqueous humor 12, the measurement of cytokine concentration such as a Vascular Endothelial Growth Factor (VEGF), and furthermore, the examination on the medicinal effect of anti-VEGF drug for suppressing the development of a new blood vessel.

The Raman scattered light spectroscopic measurement system 20 includes a light source unit 1, a polarization control unit 2, an eyepiece optical lens 3, a lens 4, a filter 5, a lens 6, and a measurement unit 7.

The light source unit 1 emits coherent light to the aqueous humor 12 of the subject eye 10. The coherent light is used as excitation light for performing spectroscopic measurement of Raman scattered light in the aqueous humor 12. In addition, the wavelength of light emitted by the light source unit 1 is 532 nm, for example, but the wavelength is not specifically limited. The light source unit 1 is only required to be a light source that emits coherent light with a predetermined wavelength, and is a Laser Diode (LD), for example.

The polarization control unit 2 is arranged between the light source unit 1 and the subject eye 10, and controls a polarization state of coherent light. The polarization control unit 2 includes, for example, a $\lambda/4$ plate 21 and a $\lambda/2$ plate 22. The polarization control unit 2 controls a polarization state of coherent light in such a manner that the light intensity of Rayleigh scattered light in the aqueous humor 12 of coherent light becomes the maximum. Specifically, by rotating high-speed axes and low-speed axes of the $\lambda/4$ plate 21 and the $\lambda/2$ plate 22 with respect to an optical axis of coherent light, the polarization control unit 2 controls a polarization state of coherent light in such a manner that the light intensity of Rayleigh scattered light becomes the maximum. Note that the polarization control unit 2 may have any configuration as long as a polarization state of coherent light can be controlled, and wave plates are not limited to the $\lambda/4$ plate 21 and the $\lambda/2$ plate 22, and the polarization control unit 2 may have a configuration including a plurality of wave plates. Furthermore, the polarization control unit 2 may include a Babinet-Soleil compensator or an acousto-optical element. In other words, the polarization control unit 2 is only required to include any one of a plurality of wave plates, a Babinet-Soleil compensator, and an acousto-optical element.

The eyepiece optical lens 3 is an eyepiece optical lens worn by or pushed against the subject eye 10, and guides coherent light emitted from the light source unit 1, into the aqueous humor 12. The eyepiece optical lens 3 may be formed of a material of a soft contact lens system such as Polyhydroxyethylmethacrylate (pHEMA) or silicone hydrogel (SHG), for example, but may be formed of a material of a hard contact lens system.

The lens 4 condenses scattered light from the aqueous humor 12.

The filter 5 includes a notch filter 51, a neutral density filter 52, and a switching plate 53 serving as a driving unit.

The notch filter 51 is a band-stop filter that selectively blocks light with a wavelength component of Rayleigh scattered light. Nevertheless, in the case of measuring Stokes Raman Scattering, in place of the notch filter 51, a filter that lets through light with a wavelength longer than that of coherent light emitted to the aqueous humor 12 may be used. In addition, in the case of measuring anti-Stokes Scattering, in place of the notch filter 51, a filter that lets through light with a wavelength shorter than that of coherent light emitted to the aqueous humor 12 may be used. In addition, in the case of measuring Raman scattered light with a known wavelength, in place of the notch filter 51, a bandpass filter that selectively lets Raman scattered light through may be used.

The neutral density filter 52 lets Rayleigh scattered light from the aqueous humor 12 through while reducing the light intensity of the Rayleigh scattered light. This is to avoid a trouble such as the saturation of the measurement unit 7 by reducing the light intensity of Rayleigh scattered light using the neutral density filter 52 in accordance with the sensitivity of the measurement unit 7, because the measurement unit 7 has the sensitivity suitable for the light intensity of Raman scattered light. Nevertheless, depending on relationship between the light intensity of Rayleigh scattered light and the sensitivity of the measurement unit 7, without using a neutral density filter, Rayleigh scattered light may be let through without being reduced. In other words, without using the neutral density filter 52, a hollow portion may be provided in the switching plate 53.

The switching plate 53 inserts and removes the notch filter 51 into and from between the subject eye 10 and the measurement unit 7.

The lens 6 condenses light having passed through the filter 5, to the measurement unit 7.

The measurement unit 7 measures a spectrum of Raman scattered light of coherent light in the aqueous humor 12. Specifically, the measurement unit 7 includes a spectral device such as a diffractive grating that disperses received light, and a detector that detects the light intensity of dispersed light with each wavelength, such as a charge-coupled device (CCD) sensor arranged in a one-dimensional array. Then, the measurement unit 7 measures light in a direction approximately orthogonal to light emitted by the light source unit 1 to the aqueous humor 12. This is because Raman scattered light can be efficiently measured since the light intensity of Rayleigh scattered light becomes the weakest in a direction approximately orthogonal to excitation light.

[Raman Scattered Light Spectroscopic Measurement Method Used by Raman Scattered Light Spectroscopic Measurement System]

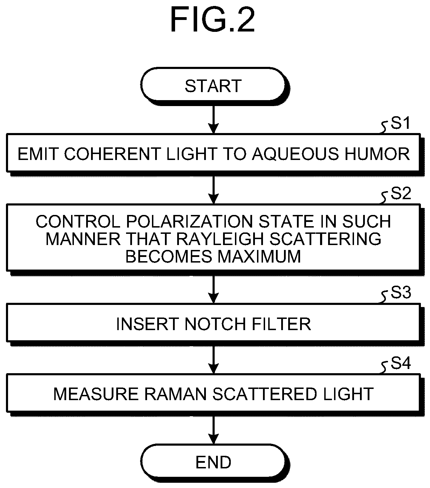

Next, a Raman scattered light spectroscopic measurement method being a light intensity measurement method to be used by the Raman scattered light spectroscopic measurement system 20 will be described. FIG. 2 is a flowchart illustrating processing to be executed by the Raman scattered light spectroscopic measurement system. Note that, in an initial state, the switching plate 53 of the filter 5 inserts the neutral density filter 52 onto an optical path extending from the subject eye 10 toward the measurement unit 7.

First of all, in a state in which the eyepiece optical lens 3 is pushed against the cornea 11 of the subject eye 10, the Raman scattered light spectroscopic measurement system 20 emits coherent light from the light source unit 1 to the aqueous humor 12 of the subject eye 10 from a direction approximately orthogonal to an eye axis of the subject eye 10 (Step S1). At this time, scattered light scattered by substances contained in the aqueous humor 12 travels in an arbitrary direction. In other words, scattered light can be received in all directions. Here, it is preferable that, by adjusting relative positions of the subject eye 10 and the Raman scattered light spectroscopic measurement system 20, the measurement unit 7 is arranged in a direction approximately conforming to the eye axis of the subject eye 10. Because the light intensity of Rayleigh scattered light becomes the weakest by performing such position adjustment, Raman scattered light can be efficiently measured.

Scattered light in the aqueous humor 12 travels from the eyepiece optical lens 3 to the outside, is condensed by the lens 4, is reduced by the neutral density filter 52 of the filter 5 in such a manner as to have the light intensity suitable for the sensitivity of the measurement unit 7, is further condensed by the lens 6, and enters the measurement unit 7. The light guided into the measurement unit 7 is dispersed by the diffractive grating or the like, and detected by the CCD sensor arranged in a one-dimensional array. Accordingly, a spectrum of scattered light in the aqueous humor 12 can be measured.

Subsequently, the polarization control unit 2 controls a polarization state of coherent light emitted from the light source unit 1, in such a manner that the light intensity of Rayleigh scattered light in the aqueous humor 12 becomes the maximum, and guides the coherent light into the subject eye 10 (Step S2). This is based on the fact that, in a polarization state in which the light intensity of Raman scattered light becomes the maximum, the light intensity of Rayleigh scattered light also becomes the maximum. Because Rayleigh scattering has the same wavelength as light emitted from the light source unit 1, it is sufficient that, in the spectrum of scattered light measured by the measurement unit 7, the light intensity of light with the same wavelength as that of light emitted from the light source unit 1 is observed. The polarization control unit 2 rotates high-speed axes and low-speed axes of the $\lambda/4$ plate 21 and the $\lambda/2$ plate 22 with respect to an optical axis of coherent light in such a manner that the light intensity of Rayleigh scattered light becomes the maximum, in the spectrum measured by the measurement unit 7. At this time, the neutral density filter 52 is arranged on the optical path extending toward the measurement unit 7, and lets part of Rayleigh scattered light through. Note that the control of the polarization control unit 2 may be executed by a control device that automatically rotates the $\lambda/4$ plate 21 and the $\lambda/2$ plate 22 in tandem with the measurement unit 7, or may be executed by a human while checking a measurement value of the measurement unit 7.

After that, by driving the switching plate 53 of the filter 5 manually or automatically using a motor, and sliding the switching plate 53 approximately vertically to an optical path extending from the subject eye 10 toward the measurement unit 7, the notch filter 51 is inserted onto this optical path (Step S3). By inserting the notch filter 51, it is possible to selectively block Rayleigh scattered light from the aqueous humor 12 that accounts for the large portion of scattered light, and let only remaining Raman scattered light through. Accordingly, it is possible to measure Raman scattered light weaker in light intensity as compared with Rayleigh scattered light.

Furthermore, Raman scattered light in the aqueous humor 12 is received by the measurement unit 7, and a spectrum of Raman scattered light is measured (Step S4). The measurement unit 7 disperses the received light by the diffractive grating, detects dispersed light with each wavelength by the CCD or the like, and measures a spectrum of Raman scattered light. The spectrum of Raman scattered light is converted into an energy difference (Raman shift) between a wavelength of excitation light and each peak wavelength of Raman scattered light. Because this Raman shift has a value corresponding to substances that causes scattering, by comprehensively evaluating the intensity of spectrum and an energy difference, analysis of concentrations of a plurality of types of substances contained in the aqueous humor 12 can be performed.

According to the above-described embodiment, by measuring a spectrum of Raman scattered light in the aqueous humor 12 by the measurement unit 7, it is possible to measure the concentration of substances contained in the aqueous humor 12. It is difficult to realize the optical path in Patent Literature 1 that directly detects light having passed through the aqueous humor 12 of the subject eye 10, but in the embodiment, because Raman scattered light is measured by the measurement unit 7 from a direction approximately orthogonal to light guided into the aqueous humor 12, it is possible to non-invasively measure the concentration of substances contained in the aqueous humor 12 of the subject eye 10.

In addition, because the amplitude of light intensity at a peak of a vibration mode appearing in the spectrum of Raman scattered light depends on a polarization state of light guided into the aqueous humor 12, it is preferable that Raman scattered light is maximized by the polarization control unit 2. Nevertheless, because the light intensity of Raman scattered light is extremely smaller as compared with another type of scattered light such as Rayleigh scattered light, it is difficult to surely capture Raman scattered light and control a polarization state. Furthermore, because the light intensity of light emitted from the light source unit 1 is restricted from the aspect of safety of the subject eye 10, it is impossible to increase the light intensity of light emitted from the light source unit 1, to increase the light intensity of Raman scattered light. Here, to maximize Raman scattered light in the aqueous humor 12, it is sufficient that the polarization state of light guided into the aqueous humor 12 is made into linear polarization vertical to a traveling direction of light. At this time, the light intensity of Rayleigh scattered light also becomes the maximum. Thus, in the embodiment, by maximizing the light intensity of Rayleigh scattered light measured by the measurement unit 7, using the polarization control unit 2, the polarization state of light in the aqueous humor 12 is made into linear polarization vertical to a traveling direction of light, and after that, the notch filter 51 that removes Rayleigh scattered light is inserted. Accordingly, according to the Raman scattered light spectroscopic measurement system 20, it is possible to perform measurement using light in the polarization state that maximizes the light intensity of Raman scattered light.

Note that, in the above-described embodiment, by maximizing Rayleigh scattered light from the aqueous humor 12 using the polarization control unit 2, and making the polarization state of light in the aqueous humor 12 into linear polarization vertical to a traveling direction of light, Raman scattered light is maximized, but the configuration is not limited to this. Making the polarization state of light in the aqueous humor 12 into linear polarization vertical to a traveling direction of light is synonymous with compensating for the influence exerted by the birefringence of the cornea 11. In other words, this corresponds to deriving an axis direction $\theta$ and a phase difference $\delta$ that constitute the birefringence of the aqueous humor 12. By controlling the polarization control unit 2 using the derived axis direction $\theta$ and the phase difference $\delta$, the polarization state of light in the aqueous humor 12 can be controlled to become an arbitrary polarization state.

First of all, in the polarization control unit 2, with respect to a traveling direction of coherent light emitted by the light source unit 1, the $\lambda/4$ plate 21 and the $\lambda/2$ plate 22 are arranged in this order. If a polarization state of coherent light emitted by the light source unit 1 is represented by a Stokes vector S, and a polarization state caused after the coherent light has passed through the $\lambda/4$ plate 21 and the $\lambda/2$ plate 22 is represented by a Stokes vector S', the following formula (1) is obtained. Note that the Stokes vectors S and S' are four-component column vectors each including zeroth to third components. In a Stokes vector, a zeroth component indicates energy of light, and first to third components indicate polarization states of light.

$$S' = M_h M_q S \qquad \cdots (1)$$

In this formula, $M_q$ and $M_h$ respectively denote Mueller matrices of the $\lambda/4$ plate 21 and the $\lambda/2$ plate 22, and can be represented by the following formulae (2) and (3). $\theta_q$ and $\theta_h$ respectively denote rotational angles of the $\lambda/4$ plate 21 and the $\lambda/2$ plate 22 from a horizontal direction.

$$M_q = \begin{pmatrix} 1 & 0 & 0 & 0 \\ 0 & \cos^2 2\theta_q & \cos 2\theta_q \sin 2\theta_q & -\sin 2\theta_q \\ 0 & \cos 2\theta_q \sin 2\theta_q & \sin^2 2\theta_q & \cos 2\theta_q \\ 0 & \sin 2\theta_q & -\cos 2\theta_q & 0 \end{pmatrix} \qquad \cdots (2)$$

$$M_h = \begin{pmatrix} 1 & 0 & 0 & 0 \\ 0 & \cos 4\theta_h & \sin 4\theta_h & 0 \\ 0 & \sin 4\theta_h & -\cos 4\theta_h & 0 \\ 0 & 0 & 0 & -1 \end{pmatrix} \qquad \cdots (3)$$

Here, if coherent light emitted by the light source unit 1 is assumed to be horizontal linearly polarized light, the Stokes vector S of the light can be represented as $S = (1100)^T$. Here, T represents transposition of a matrix element.

In addition, if a Mueller matrix representing the birefringence of a cornea is denoted by $M_b$, using an axis direction $\theta$ and a phase difference $\delta$ that constitute the birefringence of the cornea, the following formula (4) is obtained.

$$M_b = \begin{pmatrix} 1 & 0 & 0 & 0 \\ 0 & \cos^2 2\theta + \sin^2 2\theta \cos\delta & \cos 2\theta \sin 2\theta (1 - \cos\delta) & -\sin 2\theta \sin\delta \\ 0 & \cos 2\theta \sin 2\theta (1 - \cos\delta) & \sin^2 2\theta + \cos^2 2\theta \cos\delta & \cos 2\theta \sin\delta \\ 0 & \sin 2\theta \sin\delta & -\cos 2\theta \sin\delta & \cos\delta \end{pmatrix} \qquad \cdots (4)$$

Furthermore, a Mueller matrix $M_{ps}$ of scattering caused when light is scattered in a direction forming 90° with incident light in the aqueous humor 12 is represented by the following formula (5). Note that A denotes a constant serving as a factor in determining the light intensity of scattered light, with respect to excitation light intensity that does not depend on the polarization state.

$$M_{ps} = A \begin{pmatrix} 1 & -1 & 0 & 0 \\ -1 & 1 & 0 & 0 \\ 0 & 0 & 0 & 0 \\ 0 & 0 & 0 & 0 \end{pmatrix} \qquad \cdots (5)$$

Then, because light intensity I of scattered light to be detected by the measurement unit 7 is proportional to the zeroth component of a Stokes vector $S'' = M_{ps} M_b M_h M_q S$, using constants K, L, and M, the light intensity I can be represented by the following formula (6). Note that $\alpha$ is a constant appearing in a phase due to the synthesis of cos and sin, and is unrelated to subsequent optimization for maximizing the light intensity I.

$$I = K cos2\theta_q sin(4\theta_h - 2\theta_q + \alpha) + L sin2\theta_q + M \qquad \cdots (6)$$

Note that constants K, L, and M are values defined based on the "axis direction $\theta$ and the phase difference $\delta$". Therefore, by measuring a change in the light intensity I that is caused in a case where the axis direction $\theta$ and the phase difference $\delta$ are changed by the polarization control unit 2, it is possible to obtain the axis direction $\theta$ and the phase difference $\delta$ that maximize the light intensity I. In addition, as a method of maximizing the light intensity I, a change in the light intensity I that is caused in a case where the axis direction $\theta$ and the phase difference $\delta$ are changed may be measured, or the constants K, L, and M may be strictly calculated using the above-described formulae (1) to (6). Calculating the constants K, L, and M is synonymous with calculating the axis direction $\theta$ and the phase difference $\delta$, and a polarization state of coherent light in the aqueous humor 12 can be controlled to become an arbitrary polarization state.

(Modified Example 1)

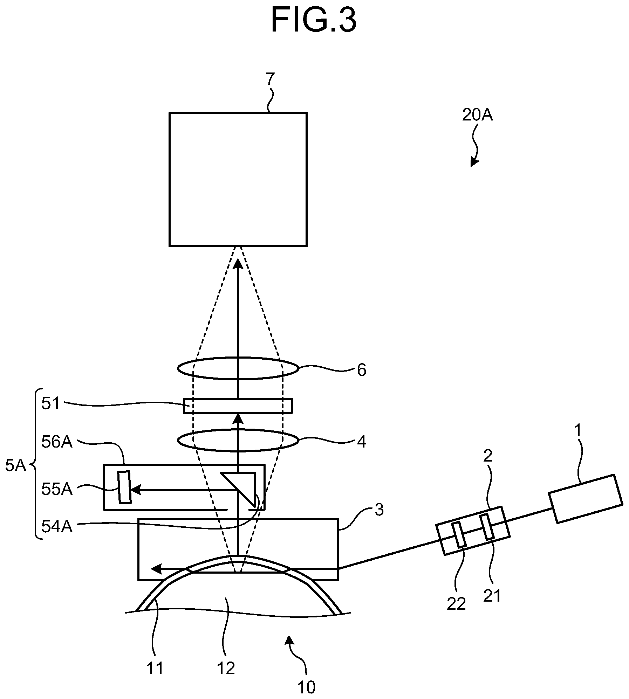

FIG. 3 is a schematic diagram illustrating a configuration of a Raman scattered light spectroscopic measurement system according to Modified Example 1. As illustrated in FIG. 3, an optical branching unit 5A of a Raman scattered light spectroscopic measurement system 20A includes a notch filter 51, a reflecting mirror 54A, a photodetector 55A, and a slide box 56A.

The material of the reflecting mirror 54A is not specifically limited as long as the reflecting mirror 54A is a mirror with high reflectivity, but it is preferable that reflectivity is constant for polarization of reflected light, and includes a metal mirror such as an aluminum, for example. Because light influenced by the birefringence of the cornea 11 enters the reflecting mirror 54A, if reflectivity is not constant for polarization, a polarization state of light in the aqueous humor 12 might fail to be accurately measured.

The photodetector 55A is a photodiode, for example.

The slide box 56A inserts and removes the reflecting mirror 54A onto and from an optical path extending from the aqueous humor 12 toward the measurement unit 7.

Next, a Raman scattered light spectroscopic measurement method to be used by the Raman scattered light spectroscopic measurement system 20A will be described. First of all, Rayleigh scattered light in the aqueous humor 12 is reflected by the reflecting mirror 54A, and detected by the photodetector 55A. By controlling the polarization control unit 2 in this state, the light intensity of Rayleigh scattered light is maximized, and the polarization state of light in the aqueous humor 12 is made into linear polarization vertical to a traveling direction of light. Then, by driving the slide box 56A and removing the reflecting mirror 54A from the optical path extending from the aqueous humor 12 toward the measurement unit 7, Raman scattered light in the aqueous humor 12 enters the measurement unit 7 via the notch filter 51.

According to the Raman scattered light spectroscopic measurement system 20A, by measuring a spectrum of Raman scattered light in the aqueous humor 12 by the measurement unit 7 using light in a polarization state that maximizes the light intensity of Raman scattered light, it is possible to non-invasively measure the concentration of substances contained in the aqueous humor 12. Furthermore, because the notch filter 51 is not moved in the Raman scattered light spectroscopic measurement system 20A, it is possible to prevent a cleaning level of an optical system from lowering due to dust or the like in an apparatus when the notch filter 51 moves.

(Modified Example 2)

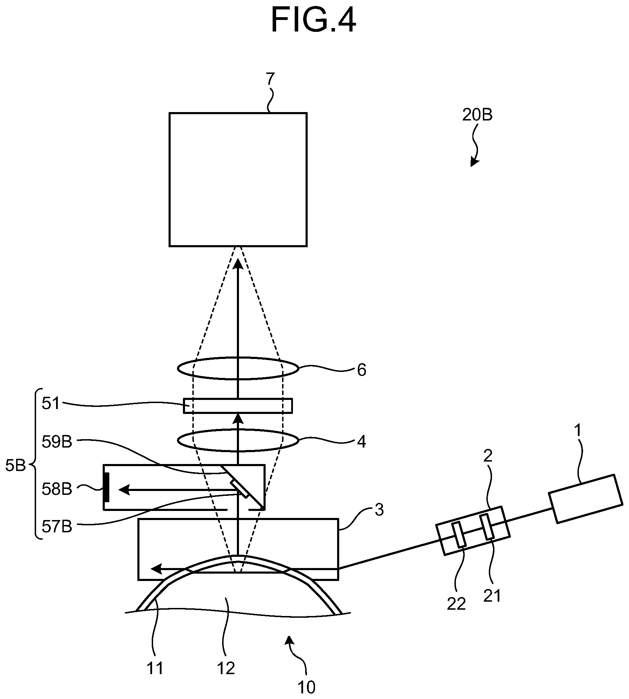

FIG. 4 is a schematic diagram illustrating a configuration of a Raman scattered light spectroscopic measurement system according to Modified Example 2. As illustrated in FIG. 4, a photodetection unit 5B of a Raman scattered light spectroscopic measurement system 20B includes a notch filter 51, a photodetector 57B, a light absorber 58B, and a slide box 59B.

The light absorber 58B absorbs light from the photodetector 57B, and prevents return light from being generated.

The slide box 59B inserts and removes the photodetector 57B onto and from an optical path extending from the aqueous humor 12 toward the measurement unit 7.

Next, a Raman scattered light spectroscopic measurement method to be used by the Raman scattered light spectroscopic measurement system 20B will be described. First of all, Rayleigh scattered light in the aqueous humor 12 is detected by the photodetector 57B. Because the photodetector 57B prevents return light returning to the subject eye 10 from the surface of the photodetector 57B, from being generated, the photodetector 57B is arranged in such a manner as to form 45°, for example, with respect to the optical axis of the subject eye 10. At this time, light reflected on the surface of the photodetector 57B is absorbed by the light absorber 58B. By controlling the polarization control unit 2 in this state, the light intensity of Rayleigh scattered light is maximized, and the polarization state of light in the aqueous humor 12 is made into linear polarization vertical to a traveling direction of light. Then, by driving the slide box 59B and removing the photodetector 57B from the optical path extending from the aqueous humor 12 toward the measurement unit 7, Raman scattered light in the aqueous humor 12 enters the measurement unit 7 via the notch filter 51.

According to the Raman scattered light spectroscopic measurement system 20B, by measuring a spectrum of Raman scattered light in the aqueous humor 12 by the measurement unit 7 using light in a polarization state that maximizes the light intensity of Raman scattered light, it is possible to non-invasively measure the concentration of substances contained in the aqueous humor 12. Furthermore, because the notch filter 51 is not moved in the Raman scattered light spectroscopic measurement system 20B, it is possible to prevent a cleaning level of an optical system from lowering due to dust or the like in an apparatus when the notch filter 51 moves. In addition, in a case where the reflecting mirror 54A is used, even if a metal mirror is used, reflectivity does not always become constant for polarization in some cases, and a measurement result might receive the influence thereof, but a reflecting mirror is not used in the Raman scattered light spectroscopic measurement system 20B. Thus, it is possible to perform measurement without receiving the influence attributed to a reflecting mirror.

In the embodiment, Modified Example 1, and Modified Example 2, the description has been given of an example in which, when a polarization state of coherent light emitted by the light source unit 1 is controlled by the polarization control unit 2, the notch filter 51 is not arranged on an optical path extending from the aqueous humor 12 toward the measurement unit 7 or the photodetector 55A or 57B, and when measurement of Raman scattered light is performed, the notch filter 51 is arranged on an optical path extending from the aqueous humor 12 toward the measurement unit 7, but the example is not limited to this. Any configuration may be employed as long as, when a polarization state of light emitted by the light source unit 1 is controlled by the polarization control unit 2, the notch filter 51 is not arranged on an optical path extending from the aqueous humor 12 toward the measurement unit 7 or the photodetector 55A or 57B, and when measurement of Raman scattered light is performed, the notch filter 51 is arranged on an optical path extending from the aqueous humor 12 toward the measurement unit 7. For example, when a polarization state of light emitted by the light source unit 1 is controlled by the polarization control unit 2, a medical service worker may remove the notch filter 51 from an optical path extending from the aqueous humor 12 toward the measurement unit 7, and when measurement of Raman scattered light is performed, a medical service worker may insert the notch filter 51 onto this optical path.

In addition, in the above-described embodiment, an example of measuring a spectrum of Raman scattered light using the Raman scattered light spectroscopic measurement system 20 has been described, but the example is not limited to this. For example, as in Patent Literature 1, a polarization state of light having passed through the aqueous humor 12 may be measured. In this case, by measuring the light intensity of Rayleigh scattered light, the axis direction $\theta$ and the phase difference $\delta$ are obtained, and by controlling the polarization control unit 2 in accordance with birefringence of the cornea 11, it becomes possible to control a polarization state of light in the aqueous humor 12 to become an arbitrary polarization state.

Reference Signs List

1 LIGHT SOURCE UNIT
2 POLARIZATION CONTROL UNIT
3 EYEPIECE OPTICAL LENS
4, 6 LENS
5 FILTER
5A OPTICAL BRANCHING UNIT
5B PHOTODETECTION UNIT
7 MEASUREMENT UNIT
10 SUBJECT EYE
11 CORNEA
12 AQUEOUS HUMOR
20, 20A, 20B RAMAN SCATTERED LIGHT SPECTROSCOPIC MEASUREMENT SYSTEM
21 $\lambda/4$ PLATE
22 $\lambda/2$ PLATE
51 NOTCH FILTER
52 NEUTRAL DENSITY FILTER
53 SWITCHING PLATE
54A REFLECTING MIRROR
55A, 57B PHOTODETECTOR
56A, 59B SLIDE BOX
58B LIGHT ABSORBER

**Claims**

1. A Raman scattered light spectroscopic measurement system (20, 20A, 20B) comprising:

a light source unit (1) configured to emit coherent light to aqueous humor (12) of a subject eye (10) from a direction approximately orthogonal to an eye axis of the subject eye (10);

a polarization control unit (2) that is arranged between the light source unit (1) and the subject eye (10), and configured to control a polarization state of the coherent light;

a measurement unit (7) configured to measure a spectrum of Raman scattered light of the coherent light in the aqueous humor (12), the measurement unit (7) being arranged in a direction approximately conforming to the eye axis of the subject eye (10); and

a notch filter (51) arranged between the subject eye (10) and the measurement unit (7) to selectively block the Rayleigh scattered light from the aqueous humor (12),

wherein the Raman scattered light spectroscopic measurement system (20, 20A, 20B) further comprises any one of the following configurations (i) to (iii):

(i) the Raman scattered light spectroscopic measurement system (20, 20A, 20B) further comprises a driving unit configured to insert and remove the notch filter (51) into and from between the subject eye (10) and the measurement unit (7),

wherein the polarization control unit (2) is configured to control the polarization state of the coherent light in such a manner that a light intensity of the Rayleigh scattered light of the coherent light in the aqueous humor (12) becomes the maximum in the spectrum measured by the measurement unit (7) when the notch filter (51) is not arranged on an optical path extending from the aqueous humor (12) toward the measurement unit (7), and

the driving unit is configured to insert the notch filter (51) onto the optical path extending from the aqueous humor (12) toward the measurement unit (7) such that the measurement unit (7) measures the spectrum of the Raman scattered light of the coherent light in the aqueous humor (12), the Rayleigh scattered light having the light intensity maximized by the polarization control unit (2);

(ii) the Raman scattered light spectroscopic measurement system (20, 20A, 20B) further comprises

a reflecting mirror (54A) configured to reflect the Rayleigh scattered light in the aqueous humor (12),

a photodetector (55A) configured to detect the Rayleigh scattered light reflected by the reflecting mirror (54A), and

a slide box (56A) configured to insert and remove the reflecting mirror (54A) onto and from an optical path extending from the aqueous humor (12) toward the measurement unit (7),

wherein the polarization control unit (2) is configured to control the polarization state of the coherent light in such a manner that a light intensity of the Rayleigh scattered light detected by the photodetector (55A) becomes the maximum, and

the slide box (56A) is configured to remove the reflecting mirror (54A) from the optical path such that the measurement unit (7) measures the spectrum of the Raman scattered light of the coherent light in the aqueous humor (12), the Rayleigh scattered light having the light intensity maximized by the polarization control unit (2); and

(iii) the Raman scattered light spectroscopic measurement system (20, 20A, 20B) further comprises

a photodetector (57B) arranged in such a manner as to form 45° with respect to an optical axis of the subject eye (10), the photodetector (57B) being configured to detect the Rayleigh scattered light in the aqueous humor (12),

a light absorber (58B) configured to absorb light from the photodetector (57B), and

a slide box (59B) configured to insert and remove the photodetector (57B) onto and from an optical path extending from the aqueous humor (12) toward the measurement unit (7),

wherein the polarization control unit (2) is configured to control the polarization state of the coherent light in such a manner that a light intensity of the Rayleigh scattered light detected by the photodetector (57B) becomes the maximum, and

the slide box (59B) is configured to remove the photodetector (57B) from the optical path such that the measurement unit (7) measures the spectrum of the Raman scattered light of the coherent light in the aqueous humor (12), the Rayleigh scattered light having the light intensity maximized by the polarization control unit (2).

2. The Raman scattered light spectroscopic measurement system (20, 20A, 20B) according to claim 1, wherein the polarization control unit (2) includes any one of a plurality of wave plates, a Babinet-Soleil compensator, and an acousto-optical element.

3. A light intensity measurement method comprising:

a light emission step of emitting coherent light to aqueous humor (12) of a subject eye (10) from a direction approximately orthogonal to an eye axis of the subject eye (10);
a polarization control step of controlling a polarization state of the coherent light and guiding the coherent light into the subject eye (10) in such a manner that a light intensity of the Rayleigh scattered light of the coherent light in the aqueous humor (12) becomes the maximum in a spectrum measured by a measurement unit (7) that is arranged in a direction approximately conforming to the eye axis of the subject eye (10);
a filter insertion step of inserting a notch filter (51) configured to selectively block the Rayleigh scattered light in the aqueous humor (12), into between the subject eye (10) and the measurement unit (7); and
a measurement step of measuring a spectrum of the Rayleigh scattered light in the aqueous humor (12) by the measurement unit (7), the Rayleigh scattered light being through the notch filter (51) and having the maximized light intensity.

**Patentansprüche**

1. Spektroskopisches Raman-Streulicht-Messsystem (20, 20A, 20B), umfassend:

eine Lichtquelleneinheit (1), die dazu angepasst ist, kohärentes Licht in Kammerwasser (12) eines Auges (10) eines Subjekts aus einer Richtung zu emittieren, die ungefähr orthogonal zu einer Augenachse des Auges (10) des Subjekts ist;
eine Polarisationssteuereinheit (2), die zwischen der Lichtquelleneinheit (1) und dem Auge (10) des Subjekts angeordnet ist, und dazu angepasst ist, einen Polarisationszustand des kohärenten Lichts zu steuern;
eine Messeinheit (7), die dazu angepasst ist, ein Spektrum von Raman-Streulicht des kohärenten Lichts in dem Kammerwasser (12) zu messen, wobei die Messeinheit (7) in einer Richtung angeordnet ist, die ungefähr der Augenachse des Auges (10) des Subjekts entspricht; und
einen Kerbfilter (51), der zwischen dem Auge (10) des Subjekts und der Messeinheit (7) angeordnet ist, um selektiv das Rayleigh-Streulicht aus dem Kammerwasser (12) zu blockieren,
wobei das spektroskopische Raman-Streulicht-Messsystem (20, 20A, 20B) ferner eine der folgenden Konfigurationen (i) bis (iii) umfasst:

(i) das spektroskopische Raman-Streulicht-Messsystem (20, 20A, 20B) umfasst ferner
eine Antriebseinheit, die dazu angepasst ist, den Kerbfilter (51) zwischen das Auge (10) des Subjekts und die Messeinheit (7) einzuführen und daraus zu entfernen,

wobei die Polarisationssteuereinheit (2) dazu angepasst ist, den Polarisationszustand des kohärenten Lichts derart zu steuern, dass eine Lichtintensität des Rayleigh-Streulichts des kohärenten Lichts in dem Kammerwasser (12) in dem von der Messeinheit (7) gemessenen Spektrum maximal wird, wenn der Kerbfilter (51) nicht auf einem optischen Pfad angeordnet ist, der sich von dem Kammerwasser (12) in Richtung der Messeinheit (7) erstreckt, und
die Antriebseinheit dazu angepasst ist, den Kerbfilter (51) auf den optischen Pfad einzuführen, der sich von dem Kammerwasser (12) in Richtung der Messeinheit (7) erstreckt, so dass die Messeinheit (7) das Spektrum des Raman-Streulichts des kohärenten Lichts in dem Kammerwasser (12) misst, wobei das Rayleigh-Streulicht die Lichtintensität aufweist, die durch die Polarisationssteuereinheit (2) maximiert wird;

(ii) das spektroskopische Raman-Streulicht-Messsystem (20, 20A, 20B) umfasst ferner

einen reflektierenden Spiegel (54A), der dazu angepasst ist, das Rayleigh-Streulicht in dem Kammerwasser (12) zu reflektieren,

einen Photodetektor (55A), der dazu angepasst ist, das Rayleigh-Streulicht zu detektieren, das durch den reflektierenden Spiegel (54A) reflektiert wird, und

einen Schiebekasten (56A), der dazu angepasst ist, den reflektierenden Spiegel (54A) auf einen optischen Pfad, der sich von dem Kammerwasser (12) in Richtung der Messeinheit (7) erstreckt, einzuführen und daraus zu entfernen,

wobei die Polarisationssteuereinheit (2) dazu angepasst ist, den Polarisationszustand des kohärenten Lichts derart zu steuern, dass eine Lichtintensität des Rayleigh-Streulichts, das durch den Photodetektor (55A) detektiert wird, maximal wird, und

der Schiebekasten (56A) dazu angepasst ist, den reflektierenden Spiegel (54A) von dem optischen Pfad zu entfernen, so dass die Messeinheit (7) das Spektrum des Raman-Streulichts des kohärenten Lichts in dem Kammerwasser (12) misst, wobei das Rayleigh-Streulicht die Lichtintensität aufweist, die durch die Polarisationssteuereinheit (2) maximiert wird; und

(iii) das spektroskopische Raman-Streulicht-Messsystem (20, 20A, 20B) umfasst ferner

einen Photodetektor (57B), der derart angeordnet ist, dass er 45° in Bezug auf eine optische Achse des Auges (10) des Subjekts bildet, wobei der Photodetektor (57B) dazu angepasst ist, das Rayleigh-Streulicht in dem Kammerwasser (12) zu detektieren,

einen Lichtabsorber (58B), der dazu angepasst ist, Licht von dem Photodetektor (57B) zu absorbieren, und

einen Schiebekasten (59B), der dazu angepasst ist, den Photodetektor (57B) auf einen optischen Pfad, der sich von dem Kammerwasser (12) in Richtung der Messeinheit (7) erstreckt, einzuführen und daraus zu entfernen,

wobei die Polarisationssteuereinheit (2) dazu angepasst ist, den Polarisationszustand des kohärenten Lichts derart zu steuern, dass eine Lichtintensität des Rayleigh-Streulichts, das durch den Photodetektor (57B) detektiert wird, maximal wird, und

der Schiebekasten (59B) dazu angepasst ist, den Photodetektor (57B) von dem optischen Pfad zu entfernen, so dass die Messeinheit (7) das Spektrum des Raman-Streulichts des kohärenten Lichts in dem Kammerwasser (12) misst, wobei das Rayleigh-Streulicht die Lichtintensität aufweist, die durch die Polarisationssteuereinheit (2) maximiert wird.

2. Spektroskopisches Raman-Streulicht-Messsystem (20, 20A, 20B) gemäß Anspruch 1, wobei die Polarisationssteuereinheit (2) eine beliebige aus einer Vielzahl von Wellenplatten, einen Babinet-Soleil-Kompensator, und ein akustooptisches Element umfasst.

3. Lichtintensitätsmessverfahren, umfassend:

einen Lichtemissionsschritt des Emittierens von kohärentem Licht in Kammerwasser (12) eines Auges (10) eines Subjekts aus einer Richtung, die ungefähr orthogonal zu einer Augenachse des Auges (10) des Subjekts ist;

einen Polarisationssteuerschritt des Steuerns eines Polarisationszustands des kohärenten Lichts und des Leitens des kohärenten Lichts in das Auge (10) des Subjekts auf eine Weise, dass eine Lichtintensität des Rayleigh-Streulichts des kohärenten Lichts in dem Kammerwasser (12) in einem Spektrum, das von einer Messeinheit (7) gemessen wird, die in einer Richtung angeordnet ist, die ungefähr der Augenachse des Auges (10) des Subjekts entspricht, maximal wird;

einen Filtereinführungsschritt des Einführens eines Kerbfilters (51), der dazu angepasst ist, selektiv das Rayleigh-Streulicht in dem Kammerwasser (12) zu blockieren, zwischen das Auge (10) des Subjekts und die Messeinheit (7); und

einen Messschritt des Messens eines Spektrums des Rayleigh-Streulichts in dem Kammerwasser (12) durch die Messeinheit (7), wobei das Rayleigh-Streulicht durch den Kerbfilter (51) geht und die maximierte Lichtintensität aufweist.

**Revendications**

1. Système de mesure spectroscopique de lumière diffusée Raman (20, 20A, 20B) comprenant :

une unité de source de lumière (1) configurée pour émettre une lumière cohérente vers l'humeur aqueuse (12) d'un œil de sujet (10) à partir d'une direction approximativement orthogonale à un axe d'œil de l'œil de sujet (10) ; une unité de commande de polarisation (2) qui est agencée entre l'unité de source de lumière (1) et l'œil de sujet (10), et configurée pour commander un état de polarisation de la lumière cohérente ; une unité de mesure (7) configurée pour mesurer un spectre de lumière diffusée Raman de la lumière cohérente dans l'humeur aqueuse (12), l'unité de mesure (7) étant agencée dans une direction se conformant approximativement à l'axe d'œil de l'œil de sujet (10) ; et un filtre coupe-bande (51) agencé entre l'œil de sujet (10) et l'unité de mesure (7) pour bloquer sélectivement la lumière diffusée Rayleigh de l'humeur aqueuse (12), où le système de mesure spectroscopique de lumière diffusée Raman (20, 20A, 20B) comprend en outre l'une quelconque des configurations (i) à (iii) suivantes :

(i) le système de mesure spectroscopique de lumière diffusée Raman (20, 20A, 20B) comprend en outre une unité d'entraînement configurée pour insérer et retirer le filtre coupe-bande (51) dans et de l'œil de sujet (10) et l'unité de mesure (7),

où l'unité de commande de polarisation (2) est configurée pour commander l'état de polarisation de la lumière cohérente de telle manière qu'une intensité lumineuse de la lumière diffusée Rayleigh de la lumière cohérente dans l'humeur aqueuse (12) devient le maximum dans le spectre mesuré par l'unité de mesure (7) lorsque le filtre coupe-bande (51) n'est pas agencé sur un chemin optique s'étendant de l'humeur aqueuse (12) vers l'unité de mesure (7), et l'unité d'entraînement est configurée pour insérer le filtre coupe-bande (51) sur le chemin optique s'étendant de l'humeur aqueuse (12) vers l'unité de mesure (7) de telle manière que l'unité de mesure (7) mesure le spectre de la lumière diffusée Raman de la lumière cohérente dans l'humeur aqueuse (12), la lumière diffusée Rayleigh ayant l'intensité lumineuse maximisée par l'unité de commande de polarisation (2) ;

(ii) le système de mesure spectroscopique de lumière diffusée Raman (20, 20A, 20B) comprend en outre

un miroir réfléchissant (54A) configuré pour réfléchir la lumière diffusée Rayleigh dans l'humeur aqueuse (12), un photodétecteur (55A) configuré pour détecter la lumière diffusée Rayleigh réfléchie par le miroir réfléchissant (54A), et une boîte coulissante (56A) configurée pour insérer et retirer le miroir réfléchissant (54A) sur et d'un chemin optique s'étendant de l'humeur aqueuse (12) vers l'unité de mesure (7), où l'unité de commande de polarisation (2) est configurée pour commander l'état de polarisation de la lumière cohérente de telle manière qu'une intensité lumineuse de la lumière diffusée Rayleigh détectée par le photodétecteur (55A) devient le maximum, et la boîte coulissante (56A) est configurée pour retirer le miroir réfléchissant (54A) du chemin optique de telle manière que l'unité de mesure (7) mesure le spectre de la lumière diffusée Raman de la lumière cohérente dans l'humeur aqueuse (12), la lumière diffusée Rayleigh ayant l'intensité lumineuse maximisée par l'unité de commande de polarisation (2) ; et

(iii) le système de mesure spectroscopique de lumière diffusée Raman (20, 20A, 20B) comprend en outre

un photodétecteur (57B) agencé de telle manière à former 45° par rapport à un axe optique de l'œil de sujet (10), le photodétecteur (57B) étant configuré pour détecter la lumière diffusée Rayleigh dans l'humeur aqueuse (12), un absorbeur de lumière (58B) configuré pour absorber la lumière provenant du photodétecteur (57B), et une boîte coulissante (59B) configurée pour insérer et retirer le photodétecteur (57B) sur et d'un chemin optique s'étendant de l'humeur aqueuse (12) vers l'unité de mesure (7),

où l'unité de commande de polarisation (2) est configurée pour commander l'état de polarisation de la lumière cohérente de telle manière qu'une intensité lumineuse de la lumière diffusée Rayleigh détectée par le photodétecteur (57B) devient le maximum, et la boîte coulissante (59B) est configurée pour retirer le photodétecteur (57B) du chemin optique de telle manière que l'unité de mesure (7) mesure le spectre de la lumière diffusée Raman de la lumière cohérente dans l'humeur aqueuse (12), la lumière diffusée Rayleigh ayant l'intensité lumineuse maximisée par l'unité de commande de polarisation (2).

**2.** Système de mesure spectroscopique de lumière diffusée Raman (20, 20A, 20B) selon la revendication 1, où l'unité de commande de polarisation (2) comprend l'une quelconque d'une pluralité de lames d'onde, un compensateur de Babinet-Soleil, et un élément acousto-optique.

**3.** Procédé de mesure d'intensité lumineuse comprenant :

une étape d'émission de lumière consistant à émettre une lumière cohérente vers l'humeur aqueuse (12) d'un œil de sujet (10) à partir d'une direction approximativement orthogonale à un axe d'œil de l'œil de sujet (10) ;
une étape de commande de polarisation consistant à commander un état de polarisation de la lumière cohérente et à guider la lumière cohérente dans l'œil de sujet (10) de telle manière qu'une intensité lumineuse de la lumière diffusée Rayleigh de la lumière cohérente dans l'humeur aqueuse (12) devient le maximum dans un spectre mesuré par une unité de mesure (7) qui est agencée dans une direction se conformant approximativement à l'axe d'œil de l'œil de sujet (10) ;
une étape d'insertion de filtre consistant à insérer un filtre coupe-bande (51) configuré pour bloquer sélectivement la lumière diffusée Rayleigh dans l'humeur aqueuse (12), dans entre l'œil de sujet (10) et l'unité de mesure (7) ; et
une étape de mesure consistant à mesurer un spectre de la lumière diffusée Rayleigh dans l'humeur aqueuse (12) par l'unité de mesure (7), la lumière diffusée Rayleigh passant à travers le filtre coupe-bande (51) et ayant l'intensité lumineuse maximisée.

# FIG.1

# FIG.2

```
      ┌─────────────┐
      │    START    │
      └──────┬──────┘
             │                          ⌐S1
      ┌──────▼──────────────────────────────┐
      │  EMIT COHERENT LIGHT TO AQUEOUS HUMOR │
      └──────┬──────────────────────────────┘
             │                          ⌐S2
      ┌──────▼──────────────────────────────┐
      │  CONTROL POLARIZATION STATE IN SUCH  │
      │  MANNER THAT RAYLEIGH SCATTERING     │
      │         BECOMES MAXIMUM              │
      └──────┬──────────────────────────────┘
             │                          ⌐S3
      ┌──────▼──────────────────────────────┐
      │         INSERT NOTCH FILTER          │
      └──────┬──────────────────────────────┘
             │                          ⌐S4
      ┌──────▼──────────────────────────────┐
      │    MEASURE RAMAN SCATTERED LIGHT     │
      └──────┬──────────────────────────────┘
             │
      ┌──────▼──────┐
      │     END     │
      └─────────────┘
```

# FIG.3

# FIG.4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005067522 A
- US 6885882 B
- WO 2020195199 A1
- US 2010245764 A1
- US 2007004975 A1